# EUROPÄISCHE PATENTSCHRIFT

(11) **EP 2 718 048 B1**
(45) Veröffentlichungstag und Bekanntmachung des Hinweises auf die Patenterteilung: **04.09.2019**
(21) Anmeldenummer: 12740469.7
(22) Anmeldetag: 08.06.2012
(51) Int. Cl.: B23B 31/02, B27C 3/00, G01N 3/40, G01N 3/58, G01N 33/46

(54) **NADEL-WECHSELKARTUSCHE, BOHRMESSGERÄT UND MONTAGE-/DEMONTAGEANORDNUNG**
EXCHANGEABLE NEEDLE CARTRIDGE, DRILLING MEASURING DEVICE, AND ASSEMBLING/DISASSEMBLING ARRANGEMENT
CARTOUCHE DE CHANGEMENT D'AIGUILLE, APPAREIL DE MESURE DE FORAGE ET DISPOSITIF DE MONTAGE/DEMONTAGE

(30) Priorität: 08.06.2011 DE 102011103636
(43) Veröffentlichungstag der Anmeldung: 16.04.2014
(73) Patentinhaber: IML-Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE)
(74) Vertreter: Drobnik, Stefanie
(86) Internationale Anmeldenummer: PCT/DE2012/000607
(87) Internationale Veröffentlichungsnummer: WO 2012/167777

(56) Entgegenhaltungen:
- DE-A- 4 122 494
- DE-A1-102005 013 752
- DE-U1- 9 117 283
- FR-A1- 2 760 842
- US-A- 3 750 343
- US-A- 4 671 105

## Beschreibung

Die nachfolgende Erfindung bezieht sich auf eine Nadel-Wechselkartusche für ein Bohrmessgerät gemäß dem Oberbegriff des Anspruchs 1 und auf ein Bohrmessgerät mit einer solchen Nadel-Wechselkartusche sowie auf eine Montage-/Demontageanordnung mit einer solchen Nadel-Wechselkartusche. Eine solche Nadel-Wechselkartusche ist aus der DE 91 17 283 U1 bekannt.

Die Gesamtheit der Gegenstände kann als Kit genutzt werden, wobei die erfindungsgemäßen Vorrichtungen es jeweils ermöglichen, das Bohrmessgerät bei dem Defekt von Komponenten, die mit dem anfälligsten Teil, nämlich der Nadel, in Verbindung stehen, schnellstmöglich und vor Ort wieder einsatzfähig zu machen.

Aus dem Stand der Technik sind beispielsweise Bohrwiderstandsmessgeräte zur Untersuchung von Bäumen und Holzbauteilen bekannt, um diese auf ihre Beschaffenheit und Defekte, etwa Fäulnis zu untersuchen.

Ein Bohrwiderstandsmessgerät zur Ermittlung des inneren Zustands von Bäumen oder aus Holz bestehenden Objekten ist etwa in der EP 09 77 653 beschrieben: Es ist im Wesentlichen einer Handbohrmaschine ähnlich und weist Mittel zum Aufzeichnen des Drehmoments der abgestützten Bohrnadel auf. Der Bohraufsatz ist mit dem Antriebsaggregat zu einer Geräteeinheit verbunden, damit die Gesamtlänge des Bohrwiderstandsmessgeräts die Länge des Bohraufsatzes nicht oder nur wenig überschreitet.

Eine Vorrichtung zur Durchführung von Bohrwiderstandsmessungen ist auch aus der DE 10 031 395 A1 bekannt, in der der Bohrwerkzeugantrieb in ein Becherteleskop einfahren gelassen wird, um eine verbesserte Führung des Bohrers bereit zu stellen.

Kommt es bei bekannten Bohrmessgeräten während der Bohrung zur Schädigung einer Nadel, so ist für den Austausch der langen und mittels verschiedener Vorrichtungen abgestützten Nadel eine komplexe und langwierige Demontage der alten Nadel erforderlich, gefolgt vom Einbau der neuen Nadel.

Ausgehend von diesem Stand der Technik ergibt sich die Aufgabe, eine Vorrichtung zum erleichterten Austauschen der Nadel für Bohrmessgeräte zu schaffen.

Diese Aufgabe wird durch die Nadel-Wechselkartusche mit den Merkmalen des Anspruchs 1 und/oder durch das Bohrmessgerät mit den Merkmalen des Anspruchs 14 und/oder durch die Montage-/Demontageanordnung mit den Merkmalen des Anspruchs 19 gelöst.

Es kann ferner geschehen, dass die Nadel in ihrem "Bohrfutter" abbricht, so dass es erforderlich wird, selbiges aus diesem oder auch aus einem anderen Grunde zu ersetzen.

Ferner wird ein verbessertes Bohrmessgerät mit den Merkmalen des Anspruchs 14 offenbart, das es ermöglicht, eine defekte Nadel oder auch eine Andockvorrichtung für die Nadel am Bohrgerät schnell und mit geringem Montageaufwand zu ersetzen.

Eine Montage-/Demontageanordnung mit den Merkmalen des Anspruchs 19 ermöglicht den leichten Austausch der Andockvorrichtung für die Nadel am Bohrgerät. Weiterbildungen der Vorrichtungen werden in den jeweiligen Unteransprüchen ausgeführt.

Eine erfindungsgemäße Nadel-Wechselkartusche für ein Bohrmessgerät umfasst ein Teleskoprohr mit einer Anzahl teleskopierbarer Rohrabschnitte, die eine Führungsvorrichtung für eine Bohrnadel bilden. Die Rohrabschnitte, die das Teleskop bilden, erstrecken sich von einem Arbeitsende des Teleskops bis zu seinem Andock-Ende: Unter dem "Arbeitsende" des Teleskoprohrs wird das Ende verstanden, an dem eine Halterung vorliegt, mittels der die Bohrnadel drehfest gehalten wird. Das Arbeitsende des Teleskoprohrs wird also zur Ausführung eines Messvorgangs an dem zu untersuchenden Objekt angelegt. Unter dem "Andock-Ende" wird hingegen das Ende des Teleskoprohrs verstanden, das an einem Antrieb andockt bzw. mit einem Antrieb gekoppelt wird, mittels dessen die Bohrnadel in Rotation versetzt wird. Um das Teleskoprohr mit einer Antriebsvorrichtung eines Bohrmessgeräts zu koppeln, weist die Nadel-Wechselkartusche eine Andockvorrichtung auf, an der das Andock-Ende des Teleskoprohrs angeordnet ist. Diese Andockvorrichtung verfügt über eine Kupplungsvorrichtung, um das Drehmoment von der Antriebsvorrichtung des Bohrmessgeräts auf das Teleskoprohr zu übertragen, und sie verfügt weiter über eine Getriebevorrichtung, um die Drehbewegung der Antriebsvorrichtung des Bohrmessgeräts in einer Linearbewegung des Teleskoprohrs zu übersetzen, sowie ferner über Mittel, durch die die Nadel-Wechselkartusche an dem Bohrmessgerät lösbar befestigt werden kann.

So wird durch die erfindungsgemäße Nadel-Wechselkartusche ermöglicht, dass die Nadel samt der sie führenden und bewegenden Komponenten ab der Schnittstelle an den Antrieb in toto ausgewechselt werden kann, wenn es zu einem Defekt kommt.

Der Aufbau der Vorrichtung ist vorteilhaft einfach gehalten.

Ferner kann die Andockvorrichtung vorteilhaft austauschbar gestaltet sein: Sie bildet dann quasi eine "Wechselandockvorrichtung", die einen Bohrfutterabschnitt mit einer Magnethalterung für das Andock-Ende des Teleskoprohres und einen Aufnahmeabschnitt, der den Bohrfutterabschnitt lösbar hält und der mit dem Bohrmessgerät verbindbar ist.

Der Aufnahmeabschnitt kann dauerhaft an dem Gehäuse, das den Antrieb für die Bohrnadel umgibt, verbleiben. Nach landläufigem Verständnis bilden ein Gehäuse, ein Antrieb und eine Aufnahmevorrichtung eine "Bohrmaschine", wobei eine derartige "Bohrmaschine" erst mit der Nadel als Bohrmessgerät einsetzbar ist.

Der "Bohrfutterabschnitt", der eine Kupplungsfunktion aufweist, ist einenends mit dem Teleskop-Rohr und anderenends mit dem Aufnahmeabschnitt lösbar verbindbar. Während der Aufnahmeabschnitt zumindest eine Befestigungsvorrichtung aufweist, mittels der der Aufnahmeabschnitt an dem Bohrmessgerät bzw. dem Gehäuse für den Antrieb befestigt wird, so dass er auch dauerhaft dort verbleiben kann, wenn gewünscht, ist der Bohrfutterabschnitt einenends mit dem Teleskoprohr und anderenends mit dem Aufnahmeabschnitt lösbar verbindbar. Somit kann dieser Bohrfutterabschnitt, in dem etwa auch ein Stück einer abgebrochenen Nadel verbleiben kann, ohne Aufwand ausgetauscht werden.

Dazu wird vorgeschlagen, dass der Bohrfutterabschnitt der Wechselandockvorrichtung über einen Schraubmechanismus mit dem Aufnahmeabschnitt verschraubbar ist. Dem Fachmann sind dazu verschiedene geeignete Gewindeformen bekannt.

Um das Austauschen der Wechselandockvorrichtung zu vereinfachen, also die Demontage und Montage der Wechselandockvorrichtung, hat der Bohrfutterabschnitt an einer Stirnseite seines von dem Aufnahmeabschnitt weg weisenden Endes Eingriffsmittel, die dazu ausgebildet sind, mit komplementären Eingriffsmitteln eines Wechselwerkzeuges für die Wechselandockvorrichtung in Eingriff gebracht zu werden.

Bei der Demontage/Montage der Wechselandockvorrichtung wird daher einfach das Wechselwerkzeug auf den Bohrfutterabschnitt der Wechselandockvorrichtung gesetzt, damit in Eingriff gebracht und die Verschraubung gelöst/angezogen.

Die an dem Bohrfutterabschnitt vorliegenden Eingriffsmittel können Bohrungen sein, wobei, um eine Übertragung des Drehmoments vom Wechselwerkzeug auf die Wechselandockvorrichtung zu ermöglichen, zumindest zwei Bohrungen, einander gegenüberliegend, vorgesehen werden sollten. Diese können etwa um 180 ° auf einem Bohrungskreis versetzt angeordnet sein. Drei oder Bohrungen sind besser sind, da so die pro Bohrung zu übertragende Kraft verringert wird. An entsprechender Stelle des Wechselwerkzeuges sind dann Passstifte vorzusehen, die in die Bohrungen eingeführt werden können, so dass das Drehmoment übertragen werden kann. Natürlich können die Eingriffsmittel auch komplexere Geometrien haben; etwa vier- oder mehrkantig oder oval in Bezug auf ihre Querschnittsform sein.

In einer weiteren Ausführungsform der Erfindung umfasst die Andockvorrichtung des Teleskoprohrs eine Magnethalterung, die lösbar mit dem Andock-Ende des Teleskoprohrs verbunden ist. Dabei ist es unerheblich, ob die Andockvorrichtung als die Wechselandockvorrichtung oder als andere, feste Andockvorrichtung ausgestaltet ist.

Mittels der Magnethalterung kann das Andock-Ende des Teleskoprohrs in der Andockvorrichtung axial zentriert geführt und gehaltert werden, um eine Ankupplung des Teleskoprohres an die Antriebsvorrichtung zu erleichtern und im verkuppelten Zustand zu unterstützen. Die Kupplungsvorrichtung zur Drehmomentübertragung auf das Teleskoprohr kann vorzugsweise eine einfache Klauenkupplung sein; aber auch eine Rastkupplung oder ein Bajonettverschluss zur Drehmoment übertragenden Kopplung von Teleskoprohr und Antriebsvorrichtung sind denkbar. Mit der Magnethalterung wird sichergestellt, dass bei dem Austausch der Nadel-Wechselkartusche am Andock-Ende keine zeitaufwändigen oder komplexen Montageschritte auszuführen sind, da durch die Magnetkraft, die bis zu 12 kg betragen kann, die erforderliche Haltekraft am Andock-Ende erzeugt wird.

Die Magnethalterung hat daher in einer geeigneten Ausführungsform eine Hohlzylindergestalt und das Andock-Ende des Teleskoprohrs kann in der Bohrung des Hohlzylinders aufgenommen sein. Dabei kann an dem Andock-Ende des Teleskoprohrs eine Zentrierscheibe vorgesehen sein, die im angedockten Zustand an der Andockvorrichtung, bzw. an der von der Andockvorrichtung umfassten Magnethalterung zur Anlage kommt. Dies ist insbesondere dann möglich, wenn in einer vorteilhaften Ausführungsform ein Rohrabschnitt mit kleinstem Außendurchmesser an dem Andock-Ende vorliegt, so dass die Rohrdurchmesser in Richtung des distalen Endes der Nadel-Wechselkartusche, von einer Anordnung mit Antrieb ausgehend, anwachsen. Grundsätzlich ist es aber auch möglich, eine umgekehrte Anordnung auszuwählen, oder eine Teleskopvorrichtung einzusetzen, die jeweils mehrere Sequenzen hat, wobei jede Sequenz aus mehreren Rohrabschnitten besteht, die in Bezug zueinander teleskopierbar angeordnet sind.

Am Arbeitsende des Teleskoprohrs hingegen liegt eine Halterung für die Bohrnadel vor, die eine entsprechende Aufnahmeöffnung aufweist und die geeigneter Weise als Schraubring ausgeführt sein kann, um mit dem Rohrabschnitt am arbeitsseitigen Ende des Teleskoprohrs verschraubt zu werden. So kann, wenn während eines Arbeitsvorgangs die Nadel-Wechselkartusche ausgetauscht werden muss, die Kartusche mit einer defekten Nadel beiseite gelegt werden, um zu einem geeigneten Zeitpunkt die Nadel leicht auszutauschen, indem sie durch Abschrauben des Schraubrings von dem Teleskoprohr entnommen wird. Die Messarbeit am Objekt muss vorteilhaft zum Austausch der Nadel nur sehr kurz unterbrochen werden.

Die Nadel-Wechselkartusche kann vorteilhaft eine Schiene umfassen, auf der die Andockvorrichtung angeordnet ist, die letztlich mit dem Teleskoprohr auf beschriebene Weise verbunden ist. Die Schiene weist Befestigungsmittel, insbesondere Eingriffsmittel auf, die etwa als eine Gabelung ausgebildet sein können, um die Schiene lösbar mit dem Bohrmessgerät zu verbinden, wenn die Nadel-Wechselkartusche in eine Benutzungsanordnung gebracht und daher mit dem Antrieb verbunden werden soll. Durch die Gestaltung als Gabelung ist es auf einfache Weise möglich, die Kartusche mit der Bohrmessvorrichtung zu verbinden, die ein Gehäuse hat, das eine entsprechende Ausnehmung aufweist, um diese Gabelung aufzunehmen. Selbstverständlich sind andere Befestigungsmittelpaare denkbar.

Das Arbeitsende des Teleskops wird in einer Ausführungsform in einem Führungsgehäuse aufgenommen, wenn es in ausgezogenem Zustand vorliegt. Das Führungsgehäuse ist ebenfalls auf der Schiene an dem von der Andockvorrichtung abgewandten Ende befestigt und stabilisiert die Anordnung, wenn die Gesamtheit aus Nadel und Teleskop in maximaler Länge vorliegt. Das Führungsgehäuse kann ferner Führungsmittel aufweisen, um die Bohrnadel des Teleskoprohres zu führen, wenn das Teleskoprohr ausfährt, bis das Arbeitsende das Führungsgehäuse erreicht ist.

In einer noch weiteren Ausführungsform der Erfindung kann die Schiene eine Seite eines Nadel-Wechselkartuschen-Gehäuses bilden, das einfach in ein übergeordnetes Gehäuse, das zu dem Bohrmessgerät gehört, eingeführt oder gesteckt werden kann. Vorteilhaft sind die Geometrie und die Länge des Bohrmessgeräte-Gehäuses so gestaltet, dass das Nadel-Wechselkartuschen-Gehäuse oder die Ausführungsform mit Schiene so darin aufgenommen werden kann, dass die Nadel in einem Nichtbetriebszustand des Bohrmessgeräts sicher darin verwahrt ist. Besonders geeignet schließt die Nadel-Wechselkartusche, etwa mittels des Deckels des Führungsgehäuses, mit dem offenen Rand des Bohrmessgeräte-Gehäuses bündig ab.

So kann vorteilhaft durch einen Handgriff das Gehäuse der Nadel-Wechselkartusche durch Lösen der Eingriffsmittel aus dem Gehäuse des Bohrmessgeräts gelöst und die Kartusche entnommen werden. Eine neue Nadel-Wechselkartusche wird eingeführt und so ist der Austausch einer defekten Nadel mit geringst möglichem Zeitaufwand und ohne komplexe Demontagevorgänge auszuführen realisierbar.

Ferner weist in einer weiteren Ausführungsform der erfindungsgemäßen Nadel-Wechselkartusche diese wenigstens ein Lager für das Teleskoprohr auf, das an einem Rohrabschnitt, der zwischen dem Arbeitsende und dem Andock-Ende liegt, angeordnet ist. Das oder die Lager ist/sind mit dem Rohrabschnitt auf der Schiene bzw. in dem Gehäuse verschiebbar, so dass das Lager beim Teleskopieren des Teleskoprohrs, also beim Überführen in die expandierte Position, mit dem entsprechenden Rohrabschnitt verfährt.

Abhängig von der Länge des Teleskoprohrs und der Anzahl der verwendeten Rohrabschnitte kann es vorteilhaft sein, mehrere Lager zu verwenden.

Besonders vorteilhaft kann die Nadel-Wechselkartusche eine Getriebevorrichtung mit einem Spindeltrieb umfassen, der mit dem Arbeitsende des Teleskoprohrs verbunden ist, um das einfache und sichere Teleskopieren mit einem bestimmten Vortrieb für die Bohrnadel sicherzustellen.

Im Grunde kann die Wechselandockvorrichtung, die eine Komponente der Nadel-Wechselkartusche sein kann, genauso für die aufgeführte Teleskoprohr-Nadelführungsvorrichtung, als auch zur Kopplung bzw. Kupplung anderer Führungsvorrichtungen für eine Bohrnadel dienen, die mit einem Bohrmessgerät betrieben werden sollen. Für den hier beschriebenen Messvorgang ist die Ausführungsform der Führungsvorrichtung, in der diese als Teleskoprohr mit mehreren teleskopierbaren Rohrabschnitten ausgebildet ist, besonders geeignet.

Wie oben dargelegt, hat die Wechselandockvorrichtung einen Bohrfutterabschnitt mit einer Magnethalterung und einen Aufnahmeabschnitt, der mit dem Bohrmessgerät verbindbar ist. Dabei ist der Bohrfutterabschnitt einenends mit der Führungsvorrichtung und anderenends mit dem Aufnahmeabschnitt lösbar verbindbar und der Aufnahmeabschnitt hat zumindest eine Befestigungsvorrichtung, die dazu ausgebildet ist, den Aufnahmeabschnitt an dem Bohrmessgerät zu befestigen.

Auch in dieser Verwendung ist der Bohrfutterabschnitt über einen Drehmechanismus mit dem Aufnahmeabschnitt verbindbar und der Bohrfutterabschnitt hat an einer Stirnseite seines von dem Aufnahmeabschnitt weg weisenden Endes Eingriffsmittel wie etwa die genannten zwei oder mehr Bohrungen, die mit komplementären Eingriffsmitteln eines Wechselwerkzeuges operativ in Eingriff gebracht werden können.

Weiter wird vorliegend ein Bohrmessgerät zur Materialbeschaffenheitsuntersuchung offenbart, das eine Antriebsvorrichtung zum Antreiben einer Bohrnadel aufweist. Das erfindungsgemäße Bohrmessgerät weist ein Gehäuse auf, in dem die Bohrnadel an einer Nadel-Wechselkartusche gemäß der vorliegenden Erfindung angeordnet ist. So ist die Bohrnadel über das Teleskoprohr und die Andockvorrichtung der Nadel-Wechselkartusche mit der Antriebsvorrichtung des Bohrmessgeräts gekoppelt und wird im Benutzungszustand vorgetrieben, indem das sich drehende Teleskoprohr ausgefahren wird.

Bei einem solchen Bohrmessgerät kann es sich um ein Bohrwiderstandsmessgerät oder ein Vorschubkraftmessgerät, das ebenfalls nach dem Funktionsprinzip einer Bohrmaschine arbeitet, handeln.

Des Weiteren wird eine Montage-//Demontageanordnung offenbart, die ein Wechselwerkzeug und eine Nadel-Wechselkartusche gemäß der vorliegenden Erfindung aufweist.

Das Wechselwerkzeug hat einen Zentralabschnitt, dessen eines Ende als Eingriffsende zum Eingriff mit der Wechselandockvorrichtung ausgebildet ist, und das stirnseitig einen zentralaxial angeordneten zylindrischen Stift aufweist. Dieser hat einen Außenumfang, der einem Innenumfang der Bohrung des Hohlzylinders der Wechselandockvorrichtung entspricht. An der Stirnseite dieses Zentralabschnitts liegen zumindest zwei Eingriffsmittel vor, die zum Eingriff mit den komplementären Eingriffsmitteln, die an der Stirnseite des Bohrfutterabschnitts der Wechselandockvorrichtung angeordnet sind, ausgebildet sind. das andere Ende des Zentralabschnitts ist als Betätigungsende ausgebildet; es erstreckt sich in einen Handgriff.

Der Handgriff soll so ausgebildet sein, dass das zum Lösen der Wechselandockvorrichtung notwendige Drehmoment mit einer möglichst kleinen Handkraft ergonomisch erzeugbar ist.

Die Eingriffsmittel am Werkzeug können Passstifte sein, und die komplementären Eingriffsmittel, die an der Stirnseite des Bohrfutterabschnitts der Wechselandockvorrichtung vorliegen, sind Bohrungen, die in Form und Durchmesser den Passstiften entsprechen. Es kann auch umgekehrt sein, so dass die Passstifte am Bohrfutterabschnitt vorliegen, wobei die komplementären Bohrungen hierbei im Wechselwerkzeug angeordnet sein müssen. Außerdem sind auch andere komplementäre Eingriffselementepaare denkbar.

Der zentralaxial angeordnete zylindrische Wellenabschnitt des Wechselwerkzeugs kann entweder als kurzer Abschnitt nur am Eingriffsende (mit dem Bohrfutterabschnitt) des Zentralabschnitts als Positionierhilfe vorliegen. Dann wird der Handgriff durch ein anderes Bauteil gebildet oder mit gebildet. Oder er kann sich zentralaxial durch den ganzen Zentralabschnitt erstrecken und am Betätigungsende aus diesem herausragen. Der Wellenabschnitt kann dann an seinem freien Ende eine Radialbohrung aufweisen, durch die ein Stab ragt und den Handgriff bildet.

Es ist auch möglich, dass der zentralaxial angeordnete zylindrische Wellenabschnitt hohl ist und eine Innenwelle aufnimmt, die sich dann zentralaxial durch den Zentralabschnitt erstreckt und am Betätigungsende auf vorstehend dargelegte Weise aus diesem herausragt, und an seinem freien Ende über die Radialbohrung mit dem Durchgangsstab verfügt und den Handgriff bildet.

Grundsätzlich kann der Handgriff auch eine andere Form aufweisen oder auf andere Weise ausgebildet sein.

Vorteilhaft wird der zentralaxial angeordnete zylindrische Wellenabschnitt an der Stirnseite des Eingriffsendes des Zentralabschnitts von einem Lagerzylinder gehalten, der so in dem Zentralabschnitt angeordnet ist, dass ein Zentriervorsprung ausgebildet wird, der in einer Montage/Demontageanordnung des Wechselwerkzeugs in der Nadelwechselkartusche passgenau über die Stufe führbar ist, die an der Andockvorrichtung dadurch gebildet wird, dass eine Ausnehmung für die Zentrierscheibe vorgesehen ist.

Wenn die Wechselandockvorrichtung als Teil einer Nadel-Wechselkartusche vorliegt, so sollte der Außenumfang des Zentralabschnitts kleiner oder gleich sein wie ein Umfang einer Öffnung, die in dem Kartuschendeckel vorliegt. Aus dieser Öffnung am Kartuschendeckel erstreckt sich üblicherweise die Bohrnadel, die an ihrem Arbeitsabschnitt von einer Halterung gehalten wird, die das "Ende" des Teleskopabschnittes bildet.

Die Öffnung kann in einer Ausführungsform eben auf eine Halterung abgestimmt sein, die als Schraubring zum Aufschrauben auf das Arbeitsende des Teleskoprohrs ausgebildet ist und die eine Bohrnadelaufnahme hat, die gerade mit der Öffnung abschließt und auf diese aufgesteckt oder -gesetzt sein kann.

Dann kann der Austausch der Nadel erfolgen, indem der Schraubring und die Bohrnadelaufnahme gelöst werden und die Nadel mit einer Zange oder anderem herausgezogen wird. Dabei bleibt das Gehäuse, das die als Teleskoprohr mit mehreren teleskopierbaren Rohrabschnitten ausgebildete Führungsvorrichtung für die Bohrnadel umgibt, an dem Bohrmessgerät, respektive an oder in dem Gehäuse, das den Antrieb umgibt, befestigt. Es ist lediglich erforderlich, am Deckel der Nadel-Wechselkartusche die Nadel aus der Bohrnadelaufnahme zu lösen, da die magnetische Befestigung der Nadel an der Kupplung durch Ziehen überwunden werden kann.

Selbiges gilt, wenn das Teleskoprohr an sich herausgenommen werden soll. Auch dann ist es lediglich erforderlich, die Halterung am Kartuschendeckel zu lösen; wenn es sich um den hier umfassten Schraubring handelt, ist dies unproblematisch und ohne Werkzeugaufwand möglich. Natürlich kann mit einer Zange nachgeholfen werden, falls der Schraubring, der die Halterung bildet zu fest sitzt.

Soll nun die Andockvorrichtung ausgetauscht werden, kann vorteilhaft einfach und geschickt auf folgende Weise vorgegangen werden, ohne dass das Gehäuse, das das Teleskoprohr umgibt, demontiert werden muss. Dazu wird, wie vorstehend dargelegt, zunächst die Nadel aus der Führungsvorrichtung genommen; dann wird das Teleskoprohr nach Abschrauben der Halterung bzw. des Schraubrings einfach aus dem umgebenden Gehäuse gezogen. Dabei ist es von Vorteil, dass die Führungsvorrichtungen, die am Teleskoprohr zu dessen Führung auf der Schiene vorgesehen sind, an den Rohrabschnitten geringeren Durchmessers vorliegen und so gestaltet sein können, dass sie problemlos durch die Öffnung am Kartuschendeckel passen. Das Teleskoprohr wird herausgezogen, optional zusammengeschoben und beiseite gelegt, und nun kommt das Wechselwerkzeug zum Einsatz. Das Eingriffsende des Wechselwerkzeugs wird voraus durch die Öffnung im Kartuschendeckel geschoben, bis die vorgesehenen Eingriffsmittel, etwa die Passstifte, mit den entsprechend komplementären Eingriffsmitteln am Bohrfutterabschnitt in Eingriff kommen. Da die Geometrien der Eingriffsmittel und ihre Anordnungen aufeinander abgestimmt sind und der Außendurchmesser des Zentralabschnitts des Wechselwerkzeugs dem Öffnungsdurchmesser der Kartuschendeckelöffnung entspricht, kann das Wechselwerkzeug leicht und sicher geführt werden, bis der Eingriff erreicht ist.

Der Zentralkörper hat dabei eine Länge, die es erlaubt, dass der Handgriff am Betätigungsende gut aus dem Gehäuse der Nadel-Wechselkartusche heraussteht und bequem betätigt, respektive gedreht werden kann. Damit wird der Bohrfutterabschnitt mit der Magnethalterung, von der Schraubverbindung des einen Aufnahmeabschnitts gelöst, der mit dem Gehäuse des Antriebs verbunden bleibt.

Das Wechselwerkzeug hält den zu demontierenden Bohrfutterabschnitt der Wechselandockvorrichtung, so dass dieser mit dem Werkzeug durch die Öffnung entnommen werden kann; ein dann zu montierender Bohrfutterabschnitt wird dann auf das Werkzeug aufgesetzt und durch die Öffnung in das Gehäuse der Nadel-Wechselkartusche eingeführt. Dabei wird auf die Positionierung geachtet und es kann der Bohrfutterabschnitt auf den Aufnahmeabschnitt bzw. auf mit einem an dem Aufnahmeabschnitt vorgesehenen zylindrischen Gewindeabschnitt verschraubt werden.

Nun wird das Wechselwerkzeug aus dem Gehäuse entnommen; das Teleskoprohr wird auseinandergezogen, wieder in das Gehäuse eingeführt und an der Wechselandockvorrichtung befestigt. Das Befestigen kann über verstärktes Eindrücken geschehen, um sicherzustellen, dass die axiale Position des Wechselwerkzeuges der für die Montage Bestimmten entspricht, d. h. das Wechselwerkzeug bestimmungsgerecht zentriert ist und an dem axialen Anschlag anliegt.

Dazu kann auch eine spezielle, auf die Öffnungsgeometrie der Kartuschengehäuseöffnung abgestimmte Drück-Kappe verwendet werden.

Abschließend wird der Schraubring der Teleskoprohr-Halterung verschraubt und die Nadel wird eingesetzt.

Das Montageverfahren ist einfach und quasi ohne Werkzeug, bis auf das genannte, durchführbar, so dass mit der Bohrnadel in Zusammenhang stehende Reparaturen jederzeit auch im Feld während der Messarbeiten in Minutenschnelle erledigbar sind.

Weitere Ausführungsformen, sowie einige der Vorteile, die mit diesen und weiteren Ausführungsformen verbunden sind, werden durch die nachfolgende ausführliche Beschreibung deutlich und besser verständlich. Unterstützend hierbei ist auch der Bezug auf die Figuren in der Beschreibung.

Dabei zeigt:
- **Fig. 1**: eine Seitenansicht eines Teleskoprohrs nach einer Ausführungsform der Erfindung mit der Schraubringhalterung für die Bohrnadel,
- **Fig. 2**: eine Teilseiten-Teilschnittansicht des Teleskoprohrs einer Ausführungsform einer Nadel-Wechselkartusche mit Schraubringhalterung für die Bohrnadel, Abstützvorrichtung, Magnethalterung und Kupplung,
- **Fig. 3**: eine perspektivische Explosionsansicht einer Nadel-Wechselkartusche gemäß einer Ausführungsform,
- **Fig. 4**: eine perspektivische Draufsicht auf ein Teleskoprohr und eine Schiene mit Andockvorrichtung und Führungsgehäuse einer Ausführungsform einer Nadel-Wechselkartusche,
- **Fig. 5**: eine perspektivische Seitenansicht eines Bohrmessgeräts mit Nadel-Wechselkartusche,

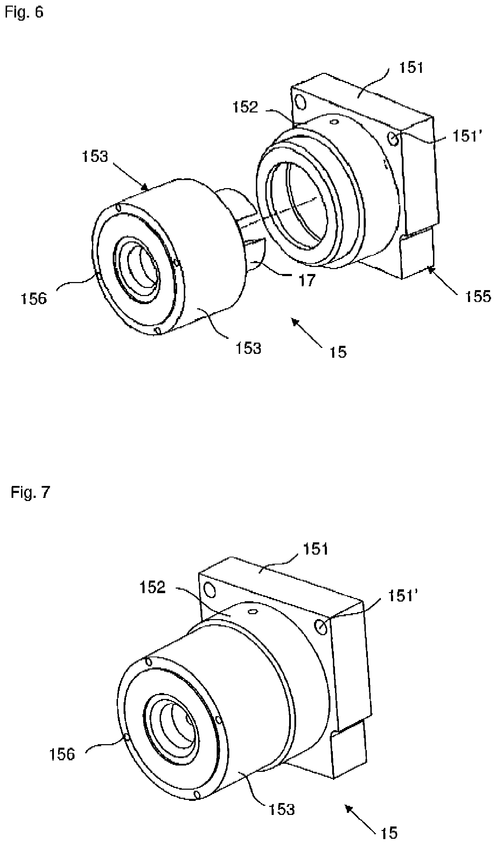

- **Fig. 6**: eine perspektivische Seitenansicht einer Wechselandockvorrichtung in nicht verbundenem Zustand,
- **Fig. 7**: eine perspektivische Seitenansicht der Wechselandockvorrichtung aus Fig. 6 in verbundenem Zustand,
- **Fig. 8**: eine perspektivische Seitenansicht der Wechselandockvorrichtung aus Fig. 6, angeordnet an einem Antriebsgehäuse,
- **Fig. 9**: eine perspektivische Seitenansicht der Wechselandockvorrichtung aus Fig. 6 in verbundenem Zustand, angeordnet an einem Antriebsgehäuse,
- **Fig. 10**: eine perspektivische Seitenansicht der Wechselandockvorrichtung aus Fig. 6, in nicht verbundenem Zustand, mit in Anordnungsposition befindlichem Teleskoprohr,
- **Fig. 11**: eine perspektivische Seitenansicht des Wechselwerkzeugs in einer Explosionsansicht,
- **Fig. 12**: eine perspektivische Seitenansicht des Wechselwerkzeugs.

Die erfindungsgemäße Nadel-Wechselkartusche ist zum Einsatz in einem Bohrmessgerät vorgesehen.

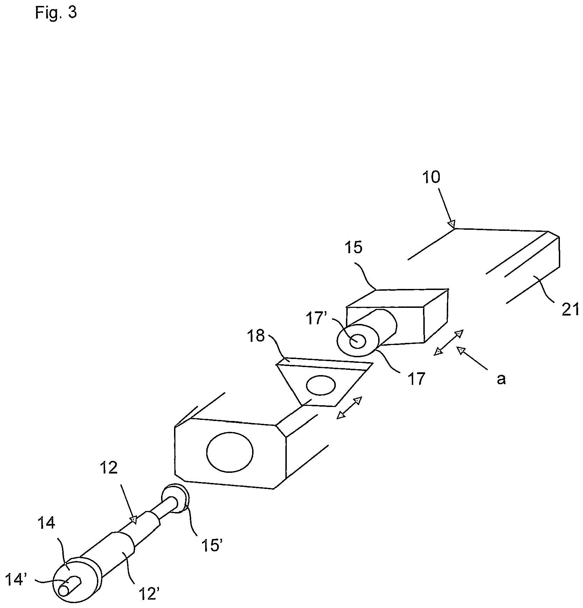

Die erfindungsgemäße Nadel-Wechselkartusche 10, siehe **Fig. 3****,** umfasst ein Teleskoprohr 12, das im Wesentlichen durch mehrere teleskopierbare Rohrabschnitte 12' gebildet ist.

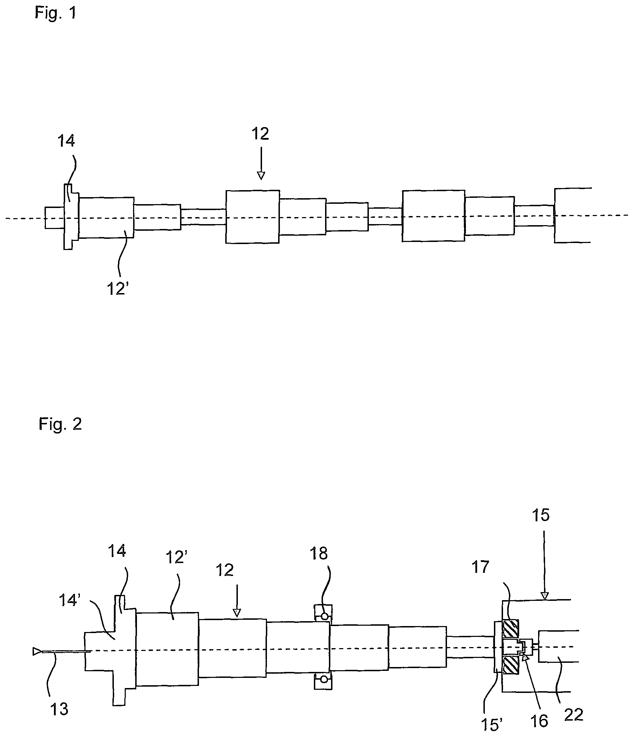

Wie in **Fig. 1** zu sehen ist, kann das Teleskoprohr 12 quasi ein aus mehreren Teleskoprohr-Sequenzen zusammengesetztes verlängertes Teleskoprohr 12 sein; in **Fig. 1** ist dabei der Rohrabschnitt 12' mit dem größten Durchmesser stets der zum Arbeitsende des Teleskoprohrs 12 gewandte, ein solcher Rohrabschnitt 12' nimmt daher auch die Bohrnadel (nicht gezeigt) mit deren Halterung 14 auf. Grundsätzlich ist es aber möglich, die Anordnung in Bezug auf die Rohrabschnitt-Durchmesser umgekehrt zu wählen.

Die Bohrnadel 13 ist, siehe **Fig. 2**, an einem Arbeitsende des Teleskoprohrs 12 mittels der Halterung 14 drehfest befestigt. Die Halterung 14, die hier als Schraubring zum Aufschrauben auf das Arbeitsende des Teleskoprohrs 12 ausgebildet ist, weist dazu eine entsprechende Bohrnadelaufnahme 14' auf.

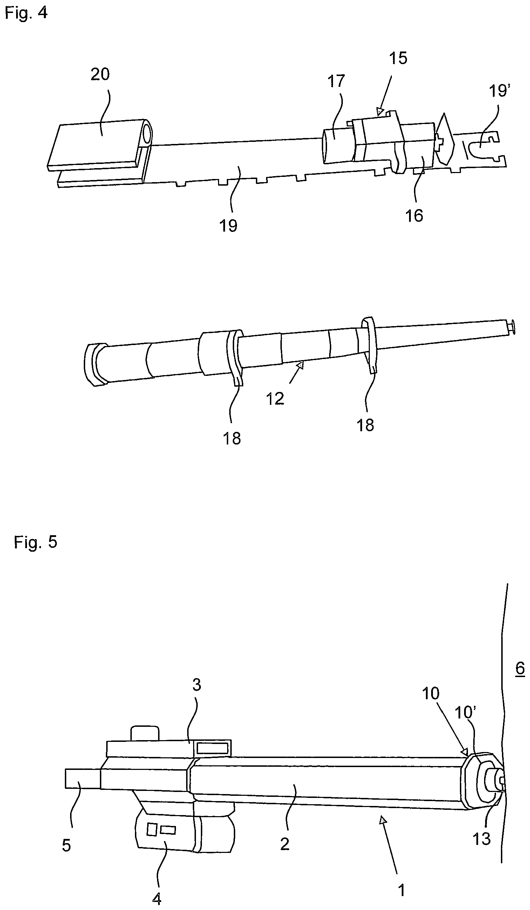

In den Figuren ist hier die Variante dargestellt, in der das Arbeitsende mit der Halterung 14 für die Bohrnadel 13 durch einen Rohrabschnitt 12' mit größtem Durchmesser gebildet wird, während der Rohrabschnitt 12' mit dem kleinstem Durchmesser das Andock-Ende des Teleskoprohrs 12 bildet, an dem das Teleskoprohr 12 mit der Andockvorrichtung 15 gekoppelt ist, die dazu dient, das Teleskoprohr 12 und damit die Bohrnadel 13 operativ mit einer Antriebsvorrichtung des Bohrwiderstandsmessgeräts 1, siehe **Fig. 5**, zu koppeln.

Mit einer von der Andockvorrichtung 15 umfassten Kupplungsvorrichtung 16 (auch in **Fig. 4** skizziert) erfolgt die Drehmomentübertragung von der Antriebsvorrichtung des Bohrwiderstandsmessgeräts 1 auf das Teleskoprohr 12. Damit die an dem Arbeitsende des Teleskoprohrs 12 befestigte Bohrnadel 13 in ein Objekt 6, siehe **Fig. 5****,** eingeführt werden kann, um dessen Bohrwiderstand oder auch die Vorschubkraft zu messen, fährt das Teleskoprohr 12 von einer zusammen geschobenen Anfangsposition auseinander, um die Bohrnadel 13 vorzutreiben. Das Teleskoprohr 12 führt entsprechend sowohl eine rotatorische Bewegung als auch eine Linearbewegung aus. Für letztere weist die Nadel-Wechselkartusche 10 beziehungsweise die Andockvorrichtung 15 eine Getriebevorrichtung auf, um die Drehbewegung der Antriebsvorrichtung des Bohrmessgeräts in eine Linearbewegung des Teleskoprohrs zu übersetzen.

Die Andockvorrichtung 15 hat eine Magnethalterung 17, mit der das Andock-Ende des Teleskoprohrs 12 lösbar verbunden werden kann, so dass das Teleskoprohr 12 mit der Bohrnadel 13 leicht ausgewechselt werden kann. Die Magnethalterung 17 ist dabei, wie in **Fig. 2** und **Fig. 3** zu sehen, als Hohlzylinder ausgeführt, in dessen Bohrung 17' das Andock-Ende des Teleskoprohrs 12 aufgenommen ist, so dass eine axiale Zentrierung des Teleskoprohrs gegeben ist und die Bohrnadel 13 mit einer Antriebsachse fluchtet. Die Magnethalterung 17 fungiert damit als eine Art Bohrfutter.

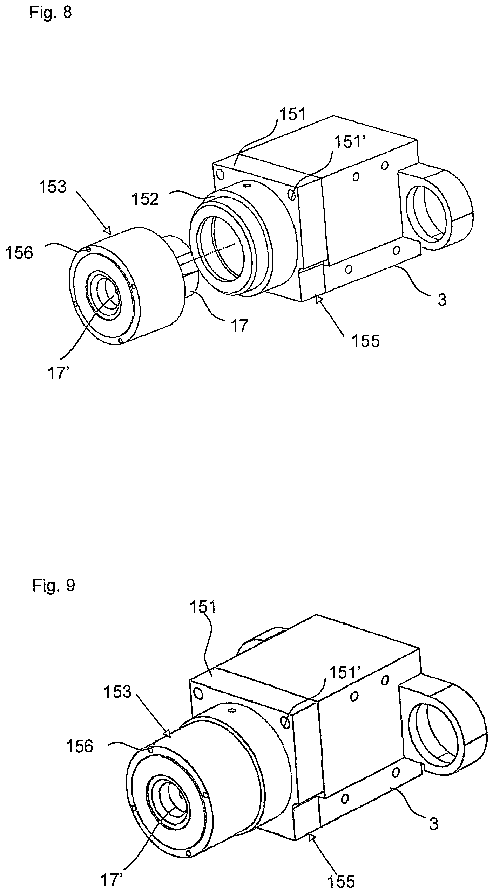

Die Andockvorrichtung 15 kann als Wechselandockvorrichtung 15 ausgeführt sein, wie sie in **Fig. 8 bis 10** gezeigt ist: Sie weist dann einen Bohrfutterabschnitt 153 auf, in dem die beschriebene mit einer Magnethalterung 17 vorliegt, und, der mit einem Aufnahmeabschnitt 155 verbunden werden kann. Dieser kann mit dem Bohrmessgerät verbunden werden, bzw. mit dessen Antriebsgehäuse 3. Dazu kann der Aufnahmeabschnitt 155 mit Bohrungen 151' ausgestattet sein, durch die eine Verschraubung mit dem Antriebsgehäuse 3 erfolgt.

Dieser Abschnitt bleibt an dem Gehäuse 3 des Motors oder Antriebs angeordnet, während der in **Fig. 8** getrennt gezeigte Bohrfutterabschitt 153 durch Verschrauben mit dem zylindrischen Gewindeabschnitt 152 des Aufnahmeabschnitts 155 verbindbar ist. Die verschraubte Anordnung ist in **Fig. 9** gezeigt.

In **Fig. 2** ist die Andockvorrichtung 15 zu sehen, in der das Andock-Ende des Teleskoprohrs 12 in der Magnethalterung 17 zentriert gehaltert wird. Die zentrierte Einführung des Andock-Endes des Teleskoprohrs 12 in die Magnethalterung 17 wird durch die Zentrierscheibe 15' unterstützt, die um das Andock-Ende des Teleskoprohrs 12 angeordnet ist und im angedockten Zustand an der Andockvorrichtung 15 bzw. der Magnethalterung 17 zur Anlage kommt. Das Andock-Ende des Teleskoprohrs 12 ist hier über eine Klauenkupplung 16 mit einer Antriebswelle 22 gekoppelt, die mit der Antriebsvorrichtung des Bohrwiderstandsmessgeräts 1 in Wirkverbindung steht. So erleichtert die Magnethalterung 17 mit der Zentrierscheibe 15' die zentrierte Einführung des Andock-Endes und damit dessen Verkupplung mit der Antriebswelle 22. Das Andock-Ende des Teleskoprohrs 12 weist dabei entweder ein Kupplungsstück, das Formschlusselemente aufweist, oder die Formschlusselemente selbst auf, die mit den entsprechenden antriebsseitigen Gegenformschlusselementen die Klauenkupplung 16 bilden.

Außer der vorbeschriebenen Magnethalterung mit Klauenkupplung sind auch andere Andockvorrichtungen denkbar, etwa in der Art eines Bajonettverschlusses oder eine Rastkupplung. Im Falle eines Bajonettverschlusses sollten die einzelnen Teleskoprohrsegmente miteinander verbunden sein, um eine den Bajonettverschluss verriegelnde Drehung des Teleskoprohres zu ermöglichen.

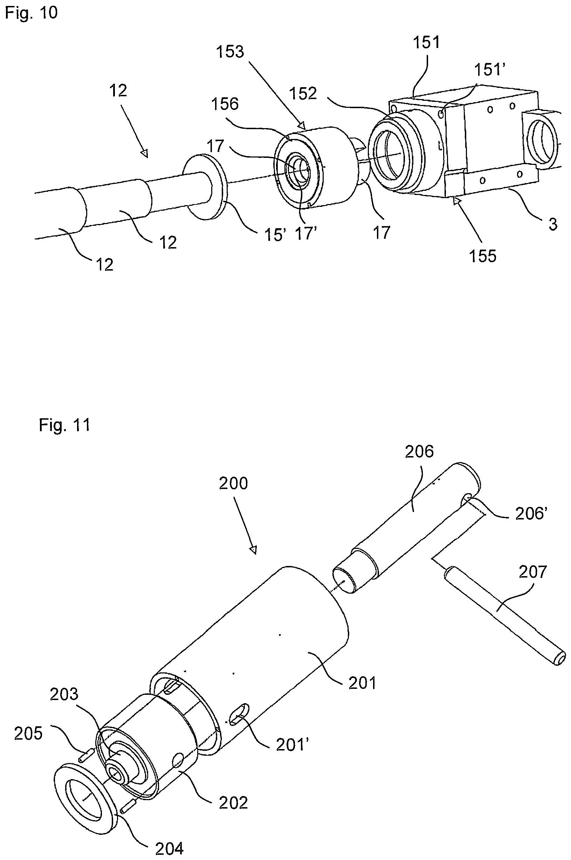

Auch der Bohrfutterabschnitt 153 der Wechselandockvorrichtung 15 kann, genau wie die nicht zum Wechseln vorgesehene Andockvorrichtung 15, über sein von dem Aufnahmeabschnitt 155 abgewandtes Ende mit dem Teleskoprohr 12 operativ gekoppelt werden. Das zur Befestigung bereits positionierte Teleskoprohr 12 ist in Fig. 10 zu sehen; die Zentrierscheibe 15' weist zu der Öffnung 17' der Magnethalterung 17, die in dem Bohrfutterabschnitt 153 vorliegt.

An der Stirnseite des Bohrfutterabschnitts 153 liegen hier vier Öffnungen 156 vor, die zur Montage oder Demontage des Bohrfutterabschnitts 153 der Wechselandockvorrichtung 15 mit den Passstiften 205 in Eingriff gebracht werden, die an der Stirnseite des Zentralabschnitts 201 des Wechselwerkzeugs 200 vorliegen.

Das Wechselwerkzeug kann zu Montage oder Demontagezwecken in das Gehäuse 21 der Nadel-Wechselkartusche 10 eingeführt und auf den Bohrfutterabschnitt 153 aufgesetzt werden, um das Lösen des Bohrfutterabschnitts 153 zu ermöglichen.

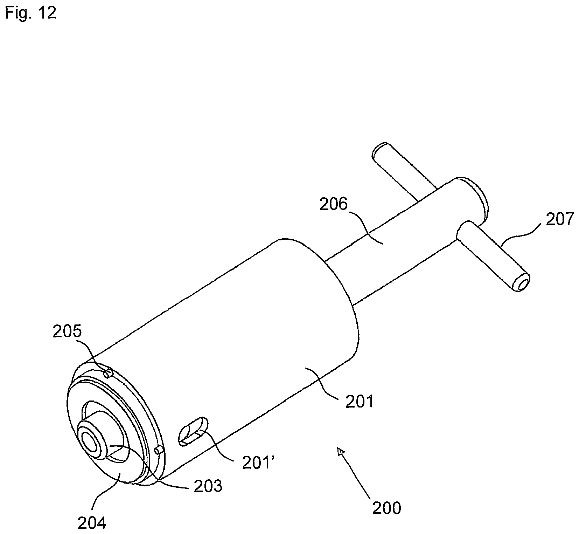

Die in **Fig. 11** **und** **12** gezeigte Ausführungsform des Werkzeugs 200 hat dabei durch den Einsatz des Lagerzylinders 202, der den zentralaxial angeordneten zylindrischen Wellenabschnitt 203 und dessen Innenwelle 206 lagert um diesen an der Stirnseite des Eingriffsendes des Werkzeugs 200 zu halten, einen Zentriervorsprung, der so ausgebildet ist, dass sie bei einer Montage/Demontageanordnung des Wechselwerkzeugs 200 in der Nadelwechselkartusche 10 passgenau über die Stufe führbar ist, die an der Andockvorrichtung 15 durch die Ausnehmung für die Zentrierscheibe 15' gebildet wird.

So bildet das Werkzeug 200 an dem Bohrfutterabschnitt 153 einen Passsitz und kann durch das Zusammenwirken der Passstifte 205 und Bohrungen 156 gedreht werden, um so den Bohrfutterabschnitt 153 von dem Aufnahmeabschnitt 155 zu lösen.

Der Zentralabschnitt des Werkzeugs hat ferner zwei sich in axialer Richtung erstreckende Langlöcher 201', die Bohrungen 202 an dem Lagerzylinder 202 überlappen und Befestigungsstifte für die Befestigung des Lagerzylinders 202 in dem Zentralabschnitt 201 aufnehmen können.

Die Befestigungsstifte können durch geeignete Maßnahmen radial nach außen drückbar sein, wodurch der Zentralabschnitt 201 und der Lagerzylinder 202 bei der Montage vorteilhaft verrastbar sind. Ferner liegt an der Stirnfläche des Lagerzylinders 202 eine Stahlscheibe 204 an, die dazu ausgebildet ist, das Einführen des Wechselwerkzeugs 200 in die korrespondierende Stufe im Bohrfutterabschnitt 153 dadurch zu erleichtern, dass die Stahlscheibe 204 vom Magnetfeld der Magnethalterung 17 angezogen wird.

Wie weiter zu sehen ist, wird der zur Betätigung des Werkzeugs erforderliche Handgriff durch den Stab 207 gebildet, der in der Radialbohrung 206' des Stabes 206 am Betätigungsende des Werkzeugs 200 angeordnet ist.

Damit das im Betrieb teleskopierende Teleskoprohr 12 geführt wird, umfasst die Nadel-Wechselkartusche 10 wenigstens ein Lager 18 **(****Fig. 2****,****3****,****4****)** das an einem der Rohrabschnitte 12' zwischen Arbeitsende und Andock-Ende angeordnet ist. Wie in **Fig. 4** zu sehen, können auch zwei Lager 18 an zwei verschiedenen Rohrabschnitten 12' vorgesehen sein.

Eine geeignete Getriebevorrichtung, um die Drehbewegung der Antriebsvorrichtung in eine Linearbewegung für das Teleskoprohr 12 zu übersetzen, ist etwa ein Spindeltrieb, der beispielsweise mit dem Arbeitsende des Teleskoprohrs 12 verbunden ist, so dass über diese Kopplung das Teleskoprohr 12 verlängert beziehungsweise nach Gebrauch wieder verkürzt werden kann.

Wie in **Fig. 4** weiter zu sehen ist, kann die Nadel-Wechselkartusche 10 eine Schiene 19 umfassen, auf der sowohl die Andockvorrichtung 15 als auch ein Führungsgehäuse 20 angeordnet ist. Die Schiene 19 weist zudem eine Gabelung 19' als Befestigungsmittel zum lösbaren Befestigen der Nadel-Wechselkartusche 10 in dem Gehäuse 2 des Bohrmessgeräts 1 auf. Die Befestigung erfolgt nach Art eines Schell- oder Klickverschlusses und bedarf keiner aufwändigen Montage oder Werkzeuge. In dem Führungsgehäuse 20 wird das Arbeitsende des Teleskoprohrs 12 mit der Halterung 14 für die Bohrnadel 13 aufgenommen, wenn es ausgezogen vorliegt.

Die Nadel-Wechselkartusche 10 kann in der in **Fig. 4** gezeigten Variante, (in montiertem Zustand) in der die Komponenten 20,25,12, auf einer Schiene 19 angeordnet sind, in das Gehäuse 2 des Bohrmessgeräts 1 eingeführt werden. Die Kartusche wird noch eleganter handhabbar, wenn wie in **Fig. 3** in Explosivansicht angedeutet, die Nadel-Wechselkartusche 10 auch ein Gehäuse 21 aufweist, dessen Boden durch die Schiene 19 gebildet wird. Vorteilhaft (siehe **Fig. 5**) ist dort die das Arbeitsende des Teleskoprohrs umgebende Seite des Führungsgehäuses 20 als ein Kartuschendeckel 10' ausgebildet, der mit dem Rand des vorderen Teils des mehrteiligen Gehäuses 2 des Bohrmessgeräts abschließt.

Das beispielhaft in **Fig. 5** gezeigte Bohrmessgerät 1 mit Nadel-Wechselkartusche 10 verfügt ferner über einen Haltegriff 5, an dem es von einem Prüfer während der Messung gehalten wird. Als Energieversorgungsmittel für den Antrieb der in einem ersten Teil des Gehäuses 2 untergebracht ist, steht der Akku 4 zur Verfügung; ein Display 3 mit Betätigungsmitteln zur Anzeige der erhaltenen Messwerte und/oder Arbeitsparametern für den Antrieb ist auf der Oberseite des ersten Teils des Gehäuses 2 untergebracht.

## Patentansprüche

1. Nadel-Wechselkartusche (10) für ein Bohrmessgerät (1), die eine als Teleskoprohr (12) mit einer Vielzahl teleskopierbarer Rohrabschnitte (12') ausgebildete Führungsvorrichtung für eine Bohrnadel (13) aufweist und die in einem Bohrmessgerät (1) einsetzbar ist,
**dadurch gekennzeichnet, dass**
sich die Vielzahl der teleskopierbaren Rohrabschnitte (12') zwischen einem Arbeitsende, an dem eine Halterung (14) zum drehfesten Halten der Bohrnadel (13) angeordnet ist, und einem Andock-Ende, an dem eine Andock-Vorrichtung (15) angeordnet ist, mittels der das Teleskoprohr (12) mit einer Antriebsvorrichtung des Bohrmessgeräts (1) koppelbar ist, erstreckt,
wobei die Andockvorrichtung (15)
- eine Kupplungsvorrichtung (16), mit der ein Drehmoment von der Antriebsvorrichtung des Bohrmessgeräts (1) auf das Teleskoprohr (12) übertragbar ist, und
- eine Getriebevorrichtung, mit der die Drehbewegung der Antriebsvorrichtung des Bohrmessgeräts (1) in eine Linearbewegung des Teleskoprohrs (12) übersetzbar ist, und
- Mittel, mit denen die Nadel-Wechselkartusche (10) lösbar an dem Bohrmessgerät (1) befestigbar ist,
umfasst.

2. Nadel-Wechselkartusche (10) nach Anspruch 1,
**dadurch gekennzeichnet, dass**
die Andockvorrichtung (15) eine Wechselandockvorrichtung (15) ist, die
- einen Bohrfutterabschnitt (153) mit einer Magnethalterung (17), und
- einen Aufnahmeabschnitt (155) aufweist, der mit dem Bohrmessgerät verbindbar ist,
wobei der Bohrfutterabschnitt (153) einenends mit der Führungsvorrichtung und anderenends mit dem Aufnahmeabschnitt (155) lösbar verbindbar ist und der Aufnahmeabschnitt (155) zumindest eine Befestigungsvorrichtung (151') aufweist, die dazu ausgebildet ist, den Aufnahmeabschnitt (155) an dem Bohrmessgerät (1) zu befestigen.

3. Nadel-Wechselkartusche (10) nach Anspruch 2,
**dadurch gekennzeichnet, dass**
der Bohrfutterabschnitt (153) der Wechselandockvorrichtung (15) über einen Schraubmechanismus mit dem Aufnahmeabschnitt (155) verschraubbar ist.

4. Nadel-Wechselkartusche nach Anspruch 2 oder 3,
**dadurch gekennzeichnet, dass**
der Bohrfutterabschnitt (153) an einer Stirnseite seines von dem Aufnahmeabschnitt (155) weg weisenden Endes Eingriffsmittel, insbesondere zumindest zwei Bohrungen (156), aufweist, die dazu ausgebildet sind, mit komplementären Eingriffsmitteln eines Wechselwerkzeuges (200) für die Wechselandockvorrichtung (15) in Eingriff gebracht zu werden.

5. Nadel-Wechselkartusche (10) nach zumindest einem der Ansprüche 1 bis 4, **dadurch gekennzeichnet, dass**
die Andock- oder die Wechselandockvorrichtung (15) eine Magnethalterung (17) umfasst, die lösbar mit dem Andock-Ende des Teleskoprohrs (12) verbunden ist und die eine axiale Zentrierung des Andock-Endes des Teleskoprohrs (12) mit der Kupplungsvorrichtung (16) bereitstellt, wobei die Kupplungsvorrichtung insbesondere eine Klauenkupplung, eine Rastkupplung oder einen Bajonettverschluss bereitstellt.

6. Nadel-Wechselkartusche (10) nach Anspruch 5,
**dadurch gekennzeichnet, dass**
die Magnethalterung (17) ein Hohlzylinder ist, in dessen Bohrung (17') das Andock-Ende des Teleskoprohrs (12) aufgenommen ist, und/oder das Andock-Ende des Teleskoprohrs (12) eine Zentrierscheibe (15') aufweist, die an der Andockvorrichtung (15), insbesondere an der Magnethalterung (17) zur Anlage kommt.

7. Nadel-Wechselkartusche (10) nach zumindest einem der Ansprüche 1 bis 6, **dadurch gekennzeichnet, dass**
die Halterung (14) als ein Schraubring (14) ausgebildet ist und eine Bohrnadelaufnahme (14') umfasst.

8. Nadel-Wechselkartusche (10) nach zumindest einem der Ansprüche 1 bis 7, **dadurch gekennzeichnet, dass**
sie eine Schiene (19) umfasst, auf der die Andockvorrichtung (15) angeordnet ist, wobei die Schiene die Mittel, insbesondere Eingriffsmittel, bevorzugt eine Gabelung (19') zur lösbaren Befestigung der Nadel-Wechselkartusche (10) an dem Bohrmessgerät (1) aufweist.

9. Nadel-Wechselkartusche (10) nach dem vorstehenden Anspruch,
**dadurch gekennzeichnet, dass**
das Arbeitsende des Teleskoprohrs (12) in einem Führungsgehäuse (20) aufgenommen ist, das auf der Schiene (19) an dem von der Andockvorrichtung (15) abgewandten Ende angeordnet ist.

10. Nadel-Wechselkartusche (10) nach Anspruch 8 oder 9,
**dadurch gekennzeichnet, dass**
die Schiene (19) eine Seite eines Nadel-Wechselkartuschen-Gehäuses (21) bildet, das in ein Gehäuse (2) des Bohrwiderstandsmessgeräts (1) einführbar und zumindest teilweise, bevorzugt vollständig in dem Gehäuse (2) des Bohrwiderstandsmessgeräts (1) aufnehmbar ist.

11. Nadel-Wechselkartusche (10) nach Anspruch 9 oder 10,
**dadurch gekennzeichnet, dass**
die das Arbeitsende des Teleskoprohrs (12) umgebende Seite des Führungsgehäuses (20) als ein Kartuschendeckel (10') ausgebildet ist, der geeignet ist, bei einer Anordnung der Nadel-Wechselkartusche (10) in dem Gehäuse (2) des Bohrwiderstandsmessgeräts (1) mit einem Rand des Gehäuses (2) abzuschließen.

12. Nadel-Wechselkartusche (10) nach zumindest einem der Ansprüche 1 bis 11, **dadurch gekennzeichnet, dass**
die Nadel-Wechselkartusche (10) zumindest ein Lager (18) für das Teleskoprohr (12) umfasst, das an einem zwischen dem Arbeitsende und dem Andock-Ende liegenden Rohrabschnitt (12') drehfest angeordnet ist, wobei das Lager (18) mit dem Rohrabschnitt (12') verschiebbar ist.

13. Nadel-Wechselkartusche (10) nach zumindest einem der Ansprüche 1 bis 12, **dadurch gekennzeichnet, dass**
die Getriebevorrichtung einen Spindeltrieb umfasst, der mit dem Arbeitsende des Teleskoprohrs (12) verbunden ist.

14. Bohrmessgerät (1) mit einer Nadel-Wechselkartusche zur Materialbeschaffenheitsuntersuchung, umfassend eine Antriebsvorrichtung zum Antreiben einer Bohrnadel (13),
**dadurch gekennzeichnet, dass**
die Nadel-Wechselkartusche eine Nadel-Wechselkartusche (10) gemäß zumindest einem der Ansprüche 1 bis 13 ist, die in einer Benutzungsanordnung des Bohrmessgeräts (1) zumindest teilweise in einem Gehäuse (2) des Bohrmessgeräts (1) aufgenommen ist,
wobei die Bohrnadel (13) über das Teleskoprohr (12) und die Andockvorrichtung (15) mit der Antriebsvorrichtung gekoppelt ist und
wobei an dem Bohrmessgerät-Gehäuse (2) Befestigungsmittel angeordnet sind, die in lösbarem Eingriff mit korrespondierenden Befestigungsmitteln der Nadel-Wechselkartusche (10) stehen.

15. Bohrmessgerät (1) nach Anspruch 14,
**dadurch gekennzeichnet, dass**
das Gehäuse (2) zumindest eine Länge und eine Geometrie derart aufweist, dass ein oder mehrere Lager (18), die das Teleskoprohr (12) lagern, in dem Gehäuse (2) verschiebbar abgestützt sind, und wobei bevorzugt das Gehäuse (2) zumindest eine Länge und eine Geometrie derart aufweist, dass die Nadel-Wechselkartusche (10), insbesondere die Schiene (19) oder das Gehäuse (21) vollständig in dem Gehäuse (2) des Bohrmessgeräts aufgenommen ist, wobei bevorzugt die das Arbeitsende des Teleskoprohrs (12) umgebende Seite des Führungsgehäuses (20) als ein Kartuschendeckel (10') ausgebildet ist, der mit einem Rand des Gehäuses (2) abschließt.

16. Bohrmessgerät (1) nach Anspruch 14 oder 15,
**dadurch gekennzeichnet, dass**
das Bohrmessgerät (1) ein Bohrwiderstandsmessgerät und/oder ein Vorschubkraft-Messgerät ist, wobei bevorzugt das Gehäuse (2) des Bohrmessgeräts (1) mehrteilig ist, wobei die Nadel-Wechselkartusche (10) in dem einen Teil und der Antrieb in einem zweiten Teil aufgenommen ist.

17. Bohrmessgerät (1) nach zumindest einem der Ansprüche 14 bis 16,
**dadurch gekennzeichnet, dass**
die Andockvorrichtung (15) eine Wechselandockvorrichtung (15) ist, die mit der Führungsvorrichtung der Nadel-Wechselkartusche (10) für die Bohrnadel (13) gekoppelt ist und die
- einen Bohrfutterabschnitt (153) mit einer Magnethalterung (17), und
- einen Aufnahmeabschnitt (155) aufweist, der mit dem Bohrmessgerät (1) verbunden ist,
wobei der Bohrfutterabschnitt (153) einenends mit der Führungsvorrichtung und anderenends mit dem Aufnahmeabschnitt (155) lösbar verbunden ist und der Aufnahmeabschnitt (155) zumindest eine Befestigungsvorrichtung (151') aufweist, die dazu ausgebildet ist, den Aufnahmeabschnitt (155) an dem Bohrmessgerät (1) zu befestigen.

18. Bohrmessgerät (1) nach Anspruch 17,
**dadurch gekennzeichnet, dass**
der Bohrfutterabschnitt (153) über einen Drehmechanismus mit dem Aufnahmeabschnitt (155) verbindbar ist, und/oder
dass der Bohrfutterabschnitt (153) an einer Stirnseite seines von dem Aufnahmeabschnitt (155) weg weisenden Endes Eingriffsmittel, insbesondere zumindest zwei Bohrungen (156) aufweist, die dazu ausgebildet sind, mit komplementären Eingriffsmitteln eines Wechselwerkzeuges (200) für die Wechselandockvorrichtung (15) in Eingriff gebracht zu werden.

19. Montage-/Demontageanordnung, die ein Wechselwerkzeug (200) und eine Nadel-Wechselkartusche (10) nach einem der Ansprüche 6 bis 13 aufweist, wobei mit dem Wechselwerkzeug (200) eine Wechselandockvorrichtung (15) der Nadel-Wechselkartusche (10) auswechselbar ist,
**dadurch gekennzeichnet, dass**
das Wechselwerkzeug (200) einen Zentralabschnitt (201) aufweist, dessen eines Ende als Eingriffsende zum Eingriff mit der Wechselandockvorrichtung (15) ausgebildet ist, und das stirnseitig einen zentralaxial angeordneten zylindrischen Wellenabschnitt (203) aufweist, der einen Außenumfang hat, der einem Innenumfang der Bohrung (17') des Hohlzylinders (17) der Wechselandockvorrichtung (15) entspricht, und an dieser Stirnseite zumindest zwei Eingriffsmittel (205) aufweist, die zum Eingriff mit den komplementären Eingriffsmitteln ausgebildet sind, die an der Stirnseite des Bohrfutterabschnitts (153) der Wechselandockvorrichtung (15) vorliegen,
und dessen anderes Ende als Betätigungsende ausgebildet ist und an dem ein Handgriff (207) angeordnet ist.

20. Montage-/Demontageanordnung nach Anspruch 19,
**dadurch gekennzeichnet, dass**
die zumindest zwei Eingriffsmittel Passstifte (205) sind, und die komplementären Eingriffsmittel, die an der Stirnseite des Bohrfutterabschnitts (153) der Wechselandockvorrichtung (15) vorliegen, Bohrungen (156) sind, und dass ein Außenumfang des Zentralabschnitts (201) kleiner oder gleich ist wie ein Umfang einer Öffnung (10"), die in dem Kartuschendeckel (10) vorliegt.

21. Montage-/Demontageanordnung nach Anspruch 19 oder 20,
**dadurch gekennzeichnet, dass**
der zentralaxial angeordnete zylindrische Wellenabschnitt (203) sich zentralaxial durch den Zentralabschnitt (201) erstreckt und am Betätigungsende aus diesem herausragt, und an seinem freien Ende eine Radialbohrung (206') aufweist, durch die ein Stab (207) ragt und den Handgriff (207) bildet,
oder, dass der zentralaxial angeordnete zylindrische Wellenabschnitt (203) hohl ist und eine Innenwelle (206) aufnimmt, die sich zentralaxial durch den Zentralabschnitt (201) erstreckt und am Betätigungsende aus diesem herausragt, und an seinem freien Ende eine Radialbohrung (206') aufweist, durch die ein Stab (207) ragt und den Handgriff (207) bildet.

22. Montage-/Demontageanordnung nach Anspruch 19 bis 21,
**dadurch gekennzeichnet, dass**
der zentralaxial angeordnete zylindrische Wellenabschnitt (203) und/oder die Innenwelle (206) an der Stirnseite des Eingriffsendes von einem Lagerzylinder (202) gehalten werden,
wobei insbesondere der Lagerzylinder (202) so in dem Zentralabschnitt (201) angeordnet ist, dass ein Zentriervorsprung ausgebildet wird, der bei einer Montage/Demontageanordnung des Wechselwerkzeugs (200) in der Nadelwechselkartusche (10) passgenau über die Stufe führbar ist, die an der Andockvorrichtung (15) durch die Ausnehmung für die Zentrierscheibe (15') gebildet wird.

## Claims

1. An exchangeable needle cartridge (10) for a drilling measuring device (1), said exchangeable needle cartridge (10) having a guiding device for a drilling needle (13), which guiding device is realized as a telescopic tube (12) with a plurality of telescopic tube portions (12'),
**characterized in that**
the plurality of telescopic tube portions (12') extends between an operating end, on which a mounting (14) is arranged for holding the drilling needle (13) in a nonrotatable manner, and a docking end, on which a docking device (15) is arranged for coupling the telescopic tube (12) with a driving device of the drilling measuring device (1), wherein the docking device (15) comprises
- a coupling device (16) for transmitting torque from the driving device of the drilling measuring device (1) to the telescopic tube (12) and
- a gear device for converting the rotational movement of the driving device of the drilling measuring device (1) into a linear movement of the telescopic tube (12) and
- means for detachably fastening the exchangeable needle cartridge (10) on the drilling measuring device (1).

2. The exchangeable needle cartridge (10) as claimed in claim 1,
**characterized in that**
the docking device (15) is an exchangeable docking device (15) which has
- a drill chuck portion (153) with a magnetic mounting (17) and
- a receiving portion (155) which is connectable to the drilling measuring device, wherein the drill chuck portion (153) is releasably connectable at one end to the guiding device and at the other end to the receiving portion (155) and the receiving portion (155) has at least one fastening device (151') which is realized for the purpose of fastening the receiving portion (155) on the drilling measuring device (1).

3. The exchangeable needle cartridge (10) as claimed in claim 2,
**characterized in that**
the drill chuck portion (153) of the exchangeable docking device (15) can be screwconnected to the receiving portion (155) by means of a screw mechanism.

4. The exchangeable needle cartridge as claimed in claim 2 or 3,
**characterized in that**
on an end face of its end pointing away from the receiving portion (155), the drill chuck portion (153) has engagement means, in particular at least two bores (156) which are realized for the purpose of being moved into engagement with complementary engagement means of an exchangeable tool (200) for the exchangeable docking device (15).

5. The exchangeable needle cartridge (10) as claimed in at least one of claims 1 to 4,
**characterized in that**
the docking or the exchangeable docking device (15) includes a magnetic mounting (17) which is releasably connected to the docking end of the telescopic tube (12) and which provides an axial centering means for the docking end of the telescopic tube (12) with the coupling device (16), wherein the coupling device provides in particular a claw coupling, a latching coupling or a bayonet closure.

6. The exchangeable needle cartridge (10) as claimed in claim 5,
**characterized in that**
the magnetic mounting (17) is a hollow cylinder, in the bore (17') of which the docking end of the telescopic tube (12) is received, and/or the docking end of the telescopic tube (12) has a centering disc (15') which abuts against the docking device (15), in particular against the magnetic mounting (17).

7. The exchangeable needle cartridge (10) as claimed in at least one of claims 1 to 6,
**characterized in that**
the mounting (14) is realized as a threaded ring (14) and includes a drilling needle receiving means (14').

8. The exchangeable needle cartridge (10) as claimed in at least one of claims 1 to 7,
**characterized in that**
said exchangeable needle cartridge (10) includes a rail (19) along which the docking device (15) is arranged, wherein the rail has the means, in particular engagement means, in a preferred manner a bifurcation (19') for the releasable fastening of the exchangeable needle cartridge (10) on the drilling measuring device (1).

9. The exchangeable needle cartridge (10) as claimed in the preceding claim,
**characterized in that**
the operating end of the telescopic tube (12) is received in a guiding housing (20) which is arranged along the rail (19) on the end which is remote from the docking device (15).

10. The exchangeable needle cartridge (10) as claimed in claim 8 or 9,
**characterized in that**
the rail (19) forms one side of an exchangeable needle cartridge housing (21) which can be inserted into a housing (2) of the drilling resistance measuring device (1) and can be received at least in part, in a preferred manner completely in the housing (2) of the drilling resistance measuring device (1).

11. The exchangeable needle cartridge (10) as claimed in claim 9 or 10,
**characterized in that**
the side of the guiding housing (20) which surrounds the operating end of the telescopic tube (12) is realized as a cartridge cover (10') which, when the exchangeable needle cartridge (10) is arranged in the housing (2) of the drilling resistance measuring device (1), is suitable to close off with an edge of the housing (2).

12. The exchangeable needle cartridge (10) as claimed in at least one of claims 1 to 11,
**characterized in that**
the exchangeable needle cartridge (10) includes at least one bearing (18) for the telescopic tube (12), said bearing being arranged in a non-rotatable manner on a tube portion (12') located between the operating end and the docking end, wherein the bearing (18) is displaceable with the tube portion (12').

13. The exchangeable needle cartridge (10) as claimed in at least one of claims 1 to 12,
**characterized in that**
the gear device includes a spindle drive which is connected to the operating end of the telescopic tube (12).

14. A drilling measuring device (1) having an exchangeable needle cartridge for testing material quality, said drilling measuring device including a driving device for driving a drilling needle (13),
**characterized in that**
the exchangeable needle cartridge is an exchangeable needle cartridge (10) as claimed in at least one of claims 1 to 13 which, in an operating arrangement of the drilling measuring device (1), is received at least in part in a housing (2) of the drilling measuring device (1),
wherein the drilling needle (13) is coupled with the driving device by means of the telescopic tube (12) and the docking device (15) and
wherein fastening means, which are engaged in a releasable manner with corresponding fastening means of the exchangeable needle cartridge (10), are arranged on the drilling measuring device housing (2).

15. The drilling measuring device (1) as claimed in claim 14,
**characterized in that**
the housing (2) has at least a length and a geometry in such a manner that one or several bearings (18), which support the telescopic tube (12), are supported so as to be displaceable in the housing (2), and wherein in a preferred manner the housing (2) has at least a length and a geometry in such a manner that the exchangeable needle cartridge (10), in particular the rail (19) or the housing (21) is received completely in the housing (2) of the drilling measuring device, wherein in a preferred manner
the side of the guiding housing (20) which surrounds the operating end of the telescopic tube (12) is realized as a cartridge cover (10') which closes off with an edge of the housing (2).

16. The drilling measuring device (1) as claimed in claim 14 or 15,
**characterized in that**
the drilling measuring device (1) is a drilling resistance measuring device and/or a moving force measuring device, wherein in a preferred manner the housing (2) of the drilling measuring device (1) is in multiple parts, wherein the exchangeable needle cartridge (10) is received in the one part and the drive in a second part.

17. The drilling measuring device (1) as claimed in at least one of claims 14 to 16,
**characterized in that**
the docking device (15) is an exchangeable docking device (15), said exchangeable docking device (15) being coupled with the guiding device of the exchangeable needle cartridge (10) for the drilling needle (13) and comprising
- a drill chuck portion (153) with a magnetic mounting (17) and
- a receiving portion (155) which is connected to the drilling measuring device (1),
wherein the drill chuck portion (153) is releasably connected at one end to the guiding device and at the other end to the receiving portion (155) and the receiving portion (155) has at least one fastening device (151') which is realized for the purpose of fastening the receiving portion (155) on the drilling measuring device (1).

18. The drilling measuring device (1) as claimed in claim 17,
**characterized in that**
the drill chuck portion (153) is connectable to the receiving portion (155) by means of a rotatable mechanism, and/or
**in that** on an end face of its end pointing away from the receiving portion (155), the drill chuck portion (153) has engagement means, in particular at least two bores (156) which are realized for the purpose of being moved into engagement with complementary engagement means of an exchangeable tool (200) for the exchangeable docking device (15).

19. Mounting/dismounting arrangement comprising an exchangeable tool (200) and an exchangeable needle cartridge (10) as claimed in one of claims 6 to 13, wherein an exchangeable docking device (15) of the exchangeable needle cartridge (10) is exchangeable by means of the exchangeable tool (200),
**characterized in that**
the exchangeable tool (200) has a central portion (201), the one end of which is realized as an engagement end for engagement with the exchangeable docking device (15), and which has on the end face a cylindrical shaft portion (203) which is arranged in a center-axial manner and has an outer circumference which corresponds to an inner circumference of the bore (17') of the hollow cylinder (17) of the exchangeable docking device (15), and on said end face has at least two engagement means (205) which are realized for engagement with the complementary engagement means which are present on the end face of the drill chuck portion (153) of the exchangeable docking device (15),
and the other end of which is realized as an actuating end and on which a handle (207) is arranged.

20. The mounting/dismounting arrangement as claimed in claim 19,
**characterized in that**
the at least two engagement means are dowel pins (205), and the complementary engagement means which are present on the end face of the drill chuck portion (153) of the exchangeable docking device (15) are bores (156), and **in that** an outer circumference of the central portion (201) is smaller than or equal to a circumference of an opening (10") which is present in the cartridge cover (10).

21. The mounting/dismounting arrangement as claimed in claim 19 or 20,
**characterized in that**
the center-axially arranged cylindrical shaft portion (203) extends center-axially through the central portion (201) and projects out of said portion at the actuating end, and on its free end has a radial bore (206') through which a bar (207) protrudes and forms the handle (207),
or **in that** the center-axially arranged cylindrical shaft portion (203) is hollow and receives an inner shaft (206) which extends center-axially through the central portion (201) and projects out of said portion at the actuating end, and on its free end has a radial bore (206') through which a bar (207) protrudes and forms the handle (207).

22. The mounting/dismounting arrangement as claimed in claims 19 to 21,
**characterized in that**
the center-axially arranged cylindrical shaft portion (203) and/or the inner shaft (206) on the end face of the engagement end are held by a bearing cylinder (202), wherein in particular the bearing cylinder (202) is arranged in the central portion (201) such that a centering projection is realized which, in the case of an arrangement of the exchangeable tool (200) for mounting/dismounting in the exchangeable needle cartridge (10), is guidable in a precisely fitting manner over the step which is formed on the docking device (15) by the recess for the centering disc (15').

## Revendications

1. Cartouche de remplacement d'aiguille (10) pour un appareil de mesure de forage (1)
qui présente un dispositif de guidage pour une aiguille de forage (13), conçu comme tube télescopique (12) possédant un grand nombre de segments de tube (12') à emmanchement télescopique, et qui est utilisable dans un appareil de mesure de forage (1),
**caractérisée en ce que,**
le grand nombre de segments de tube (12') à emmanchement télescopique s'étend entre une extrémité de travail à laquelle est disposée une monture (14) destinée à tenir l'aiguille de forage (13) solidairement en rotation et une extrémité d'introduction à laquelle est disposé un dispositif d'introduction (15) servant à accoupler le tube télescopique (12) à un dispositif d'entraînement de l'appareil de mesure de forage (1),
le dispositif d'introduction (15) comprenant
- un dispositif d'accouplement (16) servant à transmettre un couple du dispositif d'entraînement de l'appareil de mesure de forage (1) au tube télescopique (12) et
- un dispositif de transmission servant à convertir le mouvement de rotation du dispositif d'entraînement de l'appareil de mesure de forage (1) en un mouvement linéaire du tube télescopique (12) et
- des moyens servant à la fixation démontable de la cartouche de remplacement d'aiguille (10) sur l'appareil de mesure de forage (1).

2. Cartouche de remplacement d'aiguille (10) conformément à la revendication n°1,
**caractérisée en ce que**
le dispositif d'introduction (15) est un dispositif d'introduction de remplacement (15) qui présente
- une section de mandrin (153) avec un support magnétique (17) et
- une section d'emmanchement (155) qui peut être reliée à l'appareil de mesure de forage,
- la section de mandrin (153) pouvant être reliée de manière amovible d'un côté avec le dispositif de guidage et de l'autre côté avec la section d'emmanchement (155) et la section d'emmanchement (155) présentant au moins un dispositif de fixation (151') qui est conçu pour fixer la section d'emmanchement (155) sur l'appareil de mesure de forage (1).

3. Cartouche de remplacement d'aiguille (10) conformément à la revendication n°2,
**caractérisée en ce que**
la section de mandrin (153) du dispositif d'introduction de remplacement (15) peut être vissée avec la section d'emmanchement (155) par un mécanisme à vis.

4. Cartouche de remplacement d'aiguille (10) conformément à la revendication n°2 ou n°3,
**caractérisée en ce que**
la section de mandrin (153) présente, sur une face frontale de son extrémité partant de la section d'emmanchement (155), des moyens de prise, notamment au moins deux perçages (156) qui sont formés pour être mis en prise avec des moyens de prise complémentaires d'un outil de remplacement (200) pour le dispositif d'introduction de remplacement (15).

5. Cartouche de remplacement d'aiguille (10) conformément au moins à l'une des revendications n°1 ou n°4,
**caractérisée en ce que**
le dispositif d'introduction ou le dispositif d'introduction de remplacement (15) comprend un support magnétique (17) qui est relié de manière amovible à l'extrémité d'introduction du tube télescopique (12) et qui assure un centrage axial de l'extrémité d'introduction du tube télescopique (12) avec le dispositif d'accouplement (16), le dispositif d'accouplement (16) assurant en particulier un accouplement à griffes, un accouplement à enclenchement ou un verrouillage par baïonnette.

6. Cartouche de remplacement d'aiguille (10) conformément à la revendication n°5,
**caractérisée en ce que**
le support magnétique (17) est un cylindre creux, dans le perçage (17') duquel est logée l'extrémité d'introduction du tube télescopique (12), et/ou l'extrémité d'introduction du tube télescopique (12) présente un disque de centrage (15') qui vient en prise sur le dispositif d'introduction (15), en particulier sur le support magnétique (17).

7. Cartouche de remplacement d'aiguille (10) conformément au moins à l'une des revendications n°1 à n°6,
**caractérisée en ce que**
le support (14) est conçu comme une bague filetée (14) et comprend un porte-aiguille (14').

8. Cartouche de remplacement d'aiguille (10) conformément au moins à l'une des revendications n°1 à n°7,
**caractérisée en ce que**
celle-ci comprend une glissière (19) sur laquelle est disposé le dispositif d'introduction (15), la glissière présentant les moyens, notamment des moyens de prise, de préférence un embranchement (19') pour la fixation amovible de la cartouche de remplacement d'aiguille (10) sur l'appareil de mesure de forage (1).

9. Cartouche de remplacement d'aiguille (10) conformément à la revendication précédente,
**caractérisée en ce que**
l'extrémité de travail du tube télescopique (12) est emmanchée dans un boîtier de guidage (20) qui est disposé à l'extrémité détournée du dispositif d'introduction (15) sur la glissière (19).

10. Cartouche de remplacement d'aiguille (10) conformément à la revendication n°8 ou n°9,
**caractérisée en ce que**
la glissière (19) forme un côté d'un boîtier de la cartouche de remplacement d'aiguille (21), qui peut être introduit dans un boîtier (2) de l'appareil de mesure de la résistance de forage (1) et peut être emmanché au moins partiellement, de préférence intégralement dans le boîtier (2) de l'appareil de mesure de la résistance de forage (1).

11. Cartouche de remplacement d'aiguille (10) conformément à la revendication n°9 ou n°10,
**caractérisée en ce que**
le côté du boîtier de guidage (20), entourant l'extrémité de travail du tube télescopique (12), est conçu comme un chapeau de cartouche (10') qui convient comme terminaison avec un bord du boîtier (2) dans une disposition de la cartouche de remplacement d'aiguille (10) dans le boîtier (2) de l'appareil de mesure de la résistance de forage (1).

12. Cartouche de remplacement d'aiguille (10) conformément au moins à l'une des revendications n°1 à n°11,
**caractérisée en ce que**
la cartouche de remplacement d'aiguille (10) comprend au moins un palier (18) pour le tube télescopique (12), lequel est disposé solidairement en rotation sur une section tubulaire (12') située entre l'extrémité de travail et l'extrémité d'introduction, le palier (18) étant déplaçable avec la section tubulaire (12').

13. Cartouche de remplacement d'aiguille (10) conformément au moins à l'une des revendications n°1 à n°12,
**caractérisée en ce que**
le dispositif de transmission comprend une commande à vis qui est reliée à l'extrémité de travail du tube télescopique (12).

14. Appareil de mesure de forage (1) avec une cartouche de remplacement
d'aiguille pour l'analyse des propriétés de matière, comprenant un dispositif d'entraînement pour l'entraînement d'une aiguille de forage (13),
**caractérisé en ce que**
la cartouche de remplacement d'aiguille est une cartouche de remplacement d'aiguille (10) conformément au moins à l'une des revendications n°1 à n°13, qui est emmanchée dans une disposition d'utilisation de l'appareil de mesure de forage (1) au moins partiellement dans un boîtier (2) de l'appareil de mesure de forage (1),
l'aiguille de forage (13) étant reliée au dispositif d'entraînement par le tube télescopique (12) et le dispositif d'introduction (15) et
des moyens de fixation qui sont en prise amovible avec des moyens de fixation correspondants de la cartouche de remplacement d'aiguille (10), étant disposés sur le boîtier de l'appareil de mesure de forage (2).

15. Appareil de mesure de forage (1) conformément à la revendication n°14,
**caractérisé en ce que**
le boîtier (2) présente au moins une longueur et une géométrie de telle sorte qu'un ou plusieurs paliers (18) qui logent le tube télescopique (12) soient soutenus de manière déplaçable dans le boîtier (2), et le boîtier (2) présentant de préférence au moins une longueur et une géométrie de telle sorte que la cartouche de remplacement d'aiguille (10), en particulier la glissière (19) ou le boîtier (21), soit entièrement logé dans le boîtier (2) de l'appareil de mesure de forage,
le côté du boîtier de guidage (20), entourant l'extrémité de travail du tube télescopique (12), étant de préférence conçu comme un chapeau de cartouche (10') qui assure une terminaison avec un bord du boîtier (2).

16. Appareil de mesure de forage (1) conformément à la revendication n°14 ou n°15,
**caractérisé en ce que**
l'appareil de mesure de forage (1) est un appareil de mesure de la résistance de forage et/ou un appareil de mesure de la force d'avance, le boîtier (2) de l'appareil de mesure de forage (1) étant de préférence en plusieurs parties, la cartouche de remplacement d'aiguille (10) étant logée dans une partie et l'entraînement dans une deuxième partie.

17. Appareil de mesure de forage (1) conformément au moins à l'une des revendications n°14 à n°16,
**caractérisé en ce que**
le dispositif d'introduction (15) est un dispositif d'introduction de remplacement (15) qui est accouplé au dispositif de guidage de la cartouche de remplacement d'aiguille (10) pour l'aiguille de forage (13) et qui présente
- une section de mandrin (153) avec un support magnétique (17) et
- une section d'emmanchement (155) qui peut être reliée à l'appareil de mesure de forage (1),
la section de mandrin (153) étant reliée de manière amovible d'un côté avec le dispositif de guidage et de l'autre côté avec la section d'emmanchement (155), et la section d'emmanchement (155) présentant au moins un dispositif de fixation (151') qui est conçu pour fixer la section d'emmanchement (155) sur l'appareil de mesure de forage (1).

18. Appareil de mesure de forage (1) conformément à la revendication n°17,
**caractérisé en ce que**
la section de mandrin (153) peut être reliée à la section d'emmanchement (155) par un mécanisme pivotant et/ou
**que** la section de mandrin (153) présente, sur une face frontale de son extrémité partant de la section d'emmanchement (155), des moyens de prise, notamment au moins deux perçages (156) qui sont formés pour être mis en prise avec des moyens de prise complémentaires d'un outil de remplacement (200) pour le dispositif d'introduction de remplacement (15).

19. Disposition de montage/démontage qui présente un outil de remplacement (200) et une cartouche de remplacement d'aiguille (10) conformément à l'une des revendications n°6 à n°13, un dispositif d'introduction de remplacement (15) de la cartouche de remplacement d'aiguille (10) étant échangeable avec l'outil de remplacement (200),
**caractérisée en ce que**
l'outil de remplacement (200) présente une section centrale (201), dont une extrémité est conçue comme extrémité de prise pour la prise avec le dispositif d'introduction de remplacement (15) et qui présente, sur la face frontale, une section d'arbre (203) cylindrique disposée de manière centrale axiale qui a une circonférence extérieure correspondant à une circonférence intérieure du perçage (17') du cylindre creux (17) du dispositif d'introduction de remplacement (15), et qui présente, sur sa face frontale, au moins deux moyens de prise (205) qui sont conçus pour une prise avec les moyens de prise complémentaires qui se trouvent sur la face frontale de la section de mandrin (153) du dispositif d'introduction de remplacement (15),
et dont l'autre extrémité est conçue comme extrémité d'actionnement et auquel une poignée (207) est disposée.

20. Disposition de montage/démontage conformément la revendication n°19,
**caractérisée en ce que**
les au moins deux moyens de prise sont des goujons de positionnement (205) et les moyens de prise complémentaires qui se trouvent sur la face frontale de la section de mandrin (153) du dispositif d'introduction de remplacement (15) sont des perçages (156) et qu'une circonférence extérieure de la section centrale (201) est inférieure ou égale à une circonférence d'un orifice (10") qui se trouve dans le chapeau de cartouche (10).

21. Disposition de montage/démontage conformément la revendication n°19 ou n°20,
**caractérisée en ce que**
la section d'arbre (203) cylindrique disposée de manière centrale axiale s'étend sur le plan central axial par la section centrale (201) et dépasse de celle-ci à l'extrémité d'actionnement et présente, à son extrémité libre, un perçage radial (206') par lequel une tige (207) fait saillie et forme la poignée (207),
ou que la section d'arbre (203) cylindrique disposée de manière centrale axiale est creuse et loge un arbre intérieur (206) qui s'étend sur le plan central axial par la section centrale (201) et dépasse de celle-ci à l'extrémité d'actionnement et présente, à son extrémité libre, un perçage radial (206') par lequel une tige (207) fait saillie et forme la poignée (207).

22. Disposition de montage/démontage conformément la revendication n°19 à n°21,
**caractérisée en ce que**
la section d'arbre (203) cylindrique disposée de manière centrale axiale et/ou l'arbre intérieur (206) sont maintenus sur la face frontale de l'extrémité de prise par un cylindre de palier (202),
en particulier le cylindre de palier (202) étant disposé dans la section centrale (201) de façon à ce qu'une saillie de centrage soit formée, laquelle peut être guidée, dans la disposition de montage/démontage de l'outil de remplacement (200) dans la cartouche de remplacement d'aiguille (10), très précisément par le niveau qui est formé par l'évidement pour le disque de centrage (15') sur le dispositif d'introduction (15).
